# EUROPÄISCHE PATENTSCHRIFT

(11) **EP 2 513 010 B1**
(45) Veröffentlichungstag und Bekanntmachung des Hinweises auf die Patenterteilung: **30.08.2017**
(21) Anmeldenummer: 10798539.2
(22) Anmeldetag: 16.12.2010
(51) Int. Cl.: C04B 35/645

(54) **KERAMISCHER VERBUNDWERKSTOFF, BESTEHEND AUS DEN HAUPTBESTANDTEILEN ALUMINIUMOXID UND ZIRKONOXID UND EINER DISPERSOIDEN PHASE**
CERAMIC COMPOSITE MATERIAL CONSISTING OF THE PREDOMINANT COMPONENTS ALUMINA AND ZIRCONIA AND A DISPERSOIDE PHASE
MATERIAU CERAMIQUE COMPOSITE CONSTITUE DES COMPOSANTS PREDOMINANTS ALUMINE ET ZIRCONE ET D'UNE PHASE DISPRSOIDE

(30) Priorität: 16.12.2009 DE 102009054799; 16.12.2009 DE 102009054798
(43) Veröffentlichungstag der Anmeldung: 24.10.2012
(73) Patentinhaber: CeramTec GmbH, 73207 Plochingen (DE)
(72) Erfinder: KUNTZ, Meinhard, 73733 Esslingen (DE); KUNTZ, Michael, 66424 Homburg (DE); GOTTWIK, Lukas, 73092 Heiningen (DE); SCHLICHER, Kristina, 73730 Esslingen (DE); MORHARDT, Andreas, 73734 Esslingen (DE); FRIEDERICH, Kilian, 73207 Plochingen (DE); SCHNEIDER, Norbert, 73614 Schorndorf (DE)
(74) Vertreter: Uppena, Franz
(86) Internationale Anmeldenummer: PCT/EP2010/069995
(87) Internationale Veröffentlichungsnummer: WO 2011/083023

(56) Entgegenhaltungen:
- EP-A1- 2 168 936
- WO-A1-2008/132158
- WO-A1-2008/132159
- WO-A2-01/80783
- WO-A2-2008/132157
- US-A- 5 032 555
- "Zirconiumoxid" In: W. Kollenberg (Hrsg.): "Technische Keramik; Grundlagen, Werkstoffe, Verfahrenstechnik; 2. Auflage", 30. November 2009 (2009-11-30), VULKAN VERLAG, D- 45128 Essen, XP002629667, ISBN: 978-3-8027-2927-7 Seiten 231-250, Seite 234, Bild 69 (c); und erster folgender Absatz; Seite 240-->"3.2.3.4" bis Seite 242, Ende des ersten Absatzes; Seite 243-->"3.2.3.6" bis Ende von Seite 245; Seite 247-->"Medizintechnik"; Seiten 248-250; Literaturstellen (1), (15), (64) und (65).
- M. N. RAHAMAN ET AL: "Ceramics for Prosthetic Hip and Knee Joint Replacement", J. AM. CERAM. SOC. 90 (7) -2007, 1. Januar 2007 (2007-01-01), Seiten 1695-1988, XP002630077,
- A.H. DE AZA ET AL: "Slow-Crack-Growth of Zirconia-Toughened Alumina Ceramics Processed by Different Methods", J. AM. CERAM. SOC., 86 (1) 2003, 1. Januar 2003 (2003-01-01), Seiten 115-120, XP002630078,
- P.F. BECHER: "Transient Thermal Stress Behavior in ZrO2-Toughened Al2O3", J. AM. CERAM. SOC., Bd. 64, Nr. 1, 16. Mai 1980 (1980-05-16), Seiten 37-39, XP002630079,

## Beschreibung

Die vorliegende Erfindung betrifft einen aus Aluminiumoxid als keramische Matrix und darin dispergiertem Zirkonoxid bestehenden Verbundwerkstoff und zusätzlich als Nebenkomponente eine dispersoide Phase, und dessen Verwendung.

Die molekularen Strukturen von metallischen Legierungen und keramischen Werkstoffen unterscheiden sich wesentlich. In der Metallbindung kreisen die Elektronen ungeordnet und mit vergleichsweise geringer Bindungskraft um die Atomkerne. Aus diesem "lockeren" Gefüge lösen sich, beispielsweise im Körpermilieu, ständig Ionen; vielfältige chemische Reaktionen sind möglich.

In keramischen Molekülen folgen die Elektronen in der Keramikbindung exakt vorgegebenen Bahnen, den sogenannten gerichteten Elektronenorbitalen. Ihre Bindungskraft ist sehr hoch, die Moleküle sind äußerst stabil. Deshalb kommt es nicht zur Bildung von Ionen, und chemische Reaktionen sind praktisch ausgeschlossen.

Die extrem stabile Keramikbindung schließt eine plastische Verformung des Materials nahezu aus. Dies bewirkt einerseits die gewünschte extrem hohe Härte, führt jedoch auf der anderen Seite zu einer relativ hohen Sprödheit. Mit dem richtigen Werkstoffdesign kann man jedoch gleichzeitig eine hohe Härte und eine hohe Zähigkeit erreichen.

Die Materialwissenschaft unterscheidet zwischen Bruchfestigkeit und Bruchzähigkeit. Die Bruchfestigkeit bezeichnet die maximale mechanische Spannung, die ein Material aushält, ohne zu brechen. Bruchzähigkeit, oder auch Risszähigkeit, beschreibt den Widerstand eines Materials gegen einsetzendes Risswachstum. In der Medizintechnik werden bereits heute keramische Materialien eingesetzt, die eine sehr hohe Bruchfestigkeit aufweisen. Einige dieser keramischen Materialien sind zusätzlich mit einer extrem hohen Bruchzähigkeit ausgestattet. Solche Materialien können viel besser als andere Keramiken einsetzenden Rissen widerstehen und einen Rissverlauf unterbrechen.

Diese Eigenschaft beruht auf zwei Verstärkungsmechanismen. Der erste Verstärkungsmechanismus ist den eingelagerten tetragonalen Zirkonoxid-Nanopartikeln zu verdanken. Diese Partikel sind einzeln in der stabilen Aluminiumoxid-Matrix verteilt. Sie erzeugen lokale Druckspitzen im Bereich der Risse und wirken so gegen die Rissausbreitung.

Der zweite Verstärkungsmechanismus wird durch plättchenförmige Kristalle erreicht, die sich in der Oxidmischung ebenfalls vereinzelt bilden. Diese "Platelets" lenken mögliche Risse um, zerstreuen Rissenergie und bauen sie damit ab. Beide Funktionen erlauben es, mit solchen Materialien auch Komponentengeometrien zu konstruieren, die früher mit Keramik nicht zu erreichen waren.

W. Kollenberg beschreibt in seinem Lehrbuch "Technische Keramik; Grundlagen, Werkstoffe, Verfahrenstechnik; 2. Auflage" im Kapitel zu "Zirconiumoxid" verschiedene ZTA-Keramiken, bei denen die Stabilisierung der teragonalen Modifikation des Zirkoniumxoids mittels chemischen Stabilisatoren wie Y₂O₃ erfolgt.

Ähnliche ZTA-Keramiken werden auch von M. N. Rahaman, et al. unter dem Titel "Ceramics for Prosthetic Hip and Knee Joint Replacement" (J. Am. Ceram. Soc., 90 (7), 2007) die eine Aluminiumoxid-Matrix und darin dispergiertes Zirkoniumoxid aufweisen.

A. H. De Aza, et al, berichten unter dem Titel "Slow-Crack-Growth of Zirconia-Toughened Alumina Ceramics Processed by Different Methods (in J. Am Ceram. Soc., 86 (1). 2003) dass ein hoher Anteil an der tetragonalen Modifikation des Zirkoniumoxids erreicht werden kann, wenn die Zirkoniumoxid-Partikel eine bestimmte Größe aufweisen.

Auch P. F. Becher beschreibt in "Transient Thermal Stress Behavior in ZrO2-Toughened Al2O3" (in J. Am. Ceram. Soc., 64 (1), 1980) verschiedenen Aluminiumoxid-Zirkoniumoxid-Keramilken, die relative hohe Anteile an tetragonalem Zirkoniumxoid enthalten.

Die WO 2008/132158 A1 und die WO 2008/132159 A1 offenbaren keramische Werkstoffe, die neben einem hohen Anteil an Aluminiumoxid auch Zirkoniumoxid und Strontiumaluminat enthalten, sowie teilweise variabler Cr-Dotierung,

Die WO 01/80783 A2 offenbart außerdem biomedizinische Komponenten, die eine ZTA-Keramik umfasst. Das Zirkoniumoxid wird dabei mit mindestens 2,1 Mol% Yttriumoxid als chemischem Stabilisator in der tetragonalen Modifikation gehalten.

Die der vorliegenden Erfindung zugrundeliegende Aufgabe bestand darin, die Eigenschaften der bekannten keramischen Materialien weiter zu verbessern. Die vorliegende Erfindung betrifft einen keramischen Verbundwerkstoff, bestehend aus den Hauptbestandteilen Aluminiumoxid und Zirkonoxid, sowie einem oder mehreren anorganischen Zuschlagstoffen, mit denen die Eigenschaften des Verbundwerkstoffs beeinflusst werden können. Dabei bildet Aluminiumoxid die Hauptkomponente mit einem Volumengehalt von > 65 %, vorzugsweise 85 bis 90 %, das Zirkonoxid bildet die Nebenkomponente mit einem Volumengehalt zwischen 10 und 35 %. Weitere Zusätze, die nachfolgend Dispersoide genannt werden, machen einen Volumengehalt von 1 bis 10%, vorzugsweise 2 bis 8 %, besonders bevorzugt 3,5 bis 7 %, aus. Sowohl Aluminiumoxid als auch Zirkonoxid können weiterhin lösliche Bestandteile enthalten. Als lösliche Bestandteile können ein oder mehrere der folgenden Elemente vorliegen: Cr, Fe, Mg, Ti, Y, Ce, Ca, Lanthanide und/oder V. Das Zirkonoxid liegt im Ausgangszustand zu 80 bis 99 %, bevorzugt von 90 bis 99 %, bezogen auf den Geamtzirkonoxidgehalt, in der tetragonalen Phase vor. Die bekannte Phasenumwandlung des Zirkonoxids von tetragonal zu monoklin wird bei dem erfindungsgemäßen Verbundwerkstoff als Verstärkungsmechanismus genutzt, um die Risszähigkeit und die Festigkeit günstig zu beeinflussen.

Die Stabilisierung der tetragonalen Phase des Zirkonoxids erfolgt im erfindungsgemäßen Verbundwerkstoff zum überwiegenden Teil überraschenderweise nicht chemisch sondern mechanisch. Daher ist der Gehalt an anorganischen chemischen Stabilisatoren relativ zum Zirkonoxid auf Werte begrenzt, die deutlich unterhalb der im Stand der Technik normalerweise verwendeten Gehalte liegen. Der im Stand der Technik üblicherweise bevorzugt verwendete chemische Stabilisator ist Y₂O₃. Weitere bekannte Stabilisatoren sind CeO₂, CaO und MgO.

Beispiele bekannter Rezepturen für keramische Verbundwerkstoffe sind:

| **Bezeichnung** | **Mol % Y₂O₃ bezogen auf ZrO₂** |
|---|---|
| Y-TZP⁽¹⁾ | 2,8 oder 3,2 |
| ZTA⁽²⁾ | 1,3 |

| | |
|---|---|
| ⁽¹⁾Yttrium toughened Zirconia ⁽²⁾ Zirconia toughened Alumina | |

Im erfindungsgemäßen Verbundwerkstoff wird ein Stabilisatorgehalt verwendet, der deutlich niedriger ist, als die im Stand der Technik verwendeten Gehalte. Erfindungsgemäß wird dies dadurch ermöglicht, dass in dem erfindungsgemäßen Verbundwerkstoff das Zirkonoxid derart in die Aluminiumoxid-Matrix eingebettet wird, dass es durch die Einbettung in der Matrix in der metastabilen tetragonalen Phase stabilisiert wird (mechanische Stabilisierung).

Voraussetzung für die mechanische Stabilisierung ist ein Aluminiumoxidanteil von mindestens 65 Vol.-%, vorzugsweise von 65 bis 90 Vol.-%, bei einem Zirkonoxidanteil von 10 bis 35 Vol.-%. Von besonderer Bedeutung für die erfindungsgemäß überraschenderweise erzielbare mechanische Stabilisierung ist die Korngröße der Zirkonoxidpartikel im erfindungsgemäßen Verbundwerkstoff. Die Korngröße der Zirkonoxidpartikel sollte durchschnittlich 0,5 µm nicht übersteigen (gemessen nach Linienschnittverfahren). Bevorzugt für den erfindungsgemäß mechanisch stabilisierten Verbundwerkstoff sind Zirkonoxidpartikel einer Korngröße von durchschnittlich 0,1 µm bis 0,2 µm, 0,2 µm bis 0,3 µm, 0,3 µm bis 0,4 µm oder von 0,4 µm bis 0,5 µm, bevorzugt von 0,1 µm bis 0,3 µm, besonders bevorzugt von 0,15 µm bis 0,25 µm.

Der Anteil an chemischen Stabilisatoren im erfindungsgemäßen Verbundwerkstoff (Anteil jeweils relativ zum Zirkonoxidgehalt) beträgt weniger als 0,2 Mol%. Erfindungsgemäß ganz besonders bevorzugt ist ein mechanisch stabilisierter Verbundwerkstoff der keinen chemischen Stabilisator enthält.

Es ist bekannt, dass Werkstoffe, die durch die Verwendung von chemischen Stabilisatoren, insbesondere Werkstoffe, die durch Y₂O₃ stabilisiert sind, zu hydrothermaler Alterung neigen. Bei diesen Werkstoffen tritt eine spontane Phasenumwandlung in Anwesenheit von Wassermolekülen bei erhöhten Temperaturen, beispielsweise bereits bei Körpertemperatur auf. Die Ursache für diese Empfindlichkeit gegenüber Wasser bei erhöhten Temperaturen ist die Ausbildung von Sauerstoffleerstellen im Zirkonoxid-Gitter, die von Hydroxidionen besetzt werden können. Dieses Phänomen wird "hydrothermale Alterung" genannt.

Der erfindungsgemäße Verbundwerkstoff weist eine deutlich geringere Neigung zu hydrothermaler Alterung auf, als Werkstoffe, die durch die Verwendung von chemischen Stabilisatoren, insbesondere durch die Verwendung von Y₂O₃ stabilisiert sind.
Durch den reduzierten Gehalt an chemischen Stabilisatoren enthält das Zirkonoxidgitter in dem erfindungsgemäßen Verbundwerkstoff proportional weniger Sauerstoffleerstellen. Somit reagiert der erfindungsgemäße Verbundwerkstoff wesentlich weniger empfindlich auf die Anwesenheit von Wasser bei erhöhten Temperaturen als dies bei den aus dem Stand der Technik bekannten Materialien der Fall ist: der erfindungsgemäße Verbundwerkstoff neigt wesentlich weniger zu hydrothermaler Alterung.
Zusätzlich zu den Hauptbestandteilen Aluminiumoxid und Zirkonoxid enthält der erfindungsgemäße Verbundwerkstoff als Nebenkomponente eine dritte Phase. Diese dritte Phase wird nachfolgend als "dispersoide Phase" bezeichnet und wird erfindungsgemäß durch nachfolgend als "Dispersoide" bezeichnete Komponenten gebildet.
Dispersoide im Sinne der vorliegenden Erfindung sind Platelets, die inelastische Mikrodeformationen ermöglichen. Die dadurch gebildete dispersoide Phase führt überraschenderweise zu einer signifikanten Erhöhung der Risszähigkeit und Festigkeit und zwar dadurch, dass mechanische Dehnungen auf mikroskopischer Ebene, also inelastische Mikrodeformationen innerhalb der dispersoiden Phase, im Verbundwerkstoff unterstützt werden. Die Partikelgrößen der erfindungsgemäß vorgesehenen Dispersoide sind deutlich größer als die Korngrößen des erfindungsgemäß eingesetzten Aluminiumoxids bzw. Zirkonoxids, sie betragen vorzugsweise 1 bis 5 µm. Der Volumenanteil der die dritte Phase bildenden Dispersoide ist im Allgemeinen deutlich geringer als der Anteil des Zirkonoxids. Der Gehalt kann vorzugsweise bis zu 10 Vol.-% betragen. Besonders bevorzugt sind Gehalte von 2 bis 8 Vol.-%, ganz besonders bevorzugt sind Gehalte von 3 bis 6 Vol.-%.

Als Dispersoide sind erfindungsgemäß grundsätzlich alle Substanzen einsetzbar, die chemisch stabil sind und während der Herstellung des Verbundwerkstoffs durch Sintern bei hohen Temperaturen nicht im Aluminiumoxid oder im Zirkonoxid in Lösung gehen und infolge ihrer Kristallstruktur inelastische Mikrodeformationen auf mikroskopischer Ebene ermöglichen. Erfindungsgemäß möglich ist sowohl die Zugabe von Dispersoiden als auch die in-situ-Bildung der Dispersoide bei der Herstellung des erfindungsgemäßen Verbundwerkstoffs. Beispiele für erfindungsgemäß geeignete Dispersoide sind Strontiumaluminat (SrAl₁₂O₁₉) oder Lanthanaluminat (LaAl₁₁O₁₈).

Die dispersoide Phase hat die Funktion, auf mikroskopischer Ebene inhomogene Dehnungen der Hauptbestandteile Aluminiumoxid und Zirkonoxid zu akkomodieren. Der Begriff "inhomogene mikroskopische Dehnung" dient zur Unterscheidung von der makroskopischen homogenen Dehnung des Materials, beispielsweise durch Wärmedehnung oder durch eine von außen angelegte mechanische Spannung. Die inhomogene mikroskopische Dehnung beschreibt lokale Ereignisse, die auf der Größenebene der Kristallite stattfinden. Insbesondere sind solche Dehnungen gemeint, die bei entsprechender Belastung des erfindungsgemäßen Verbundwerkstoffs und der dadurch initiierten und erfindungsgemäß gewollten Phasenumwandlung des Zirkonoxids von der tetragonalen in die monokline Phase ausgelöst werden. Die Phasenumwandlung von tetragonal zu monoklin ist mit einer Volumenzunahme von etwa 4 % verbunden und in der Literatur, beispielsweise in D.J. Green, Transformation Toughening of Ceramics, CRC Press Florida, 1989, ISBN 0-8493-6594-5, umfassend beschrieben. Sie wird durch hohe lokale Zugspannungen, beispielsweise in der Nähe von Werkstoffdefekten, ausgelöst und bewirkt eine Steigerung der Risszähigkeit des Werkstoffs, die so genannte Umwandlungsverstärkung. Durch die Phasenumwandlung einzelner Zirkonoxidkristallite wird deren Umgebung stark gedehnt. Um diesen Vorgang im erfindungsgemäßen Verbundwerkstoff für die Verbesserung der Werkstoffeigenschaften in optimaler Art und Weise ausnutzen zu können, werden die lokalen Dehnungen in der Umgebung der umgewandelten Zirkonoxidkristallite durch den erfindungsgemäßen Einsatz der dispersoiden Phase akkomodiert.

Unter "Akkomodation" im Sinne der vorliegenden Erfindung ist folgender Mechanismus zu verstehen: Die erfindungsgemäß vorgesehenen Dispersoide führen dazu, dass die bei entsprechender Belastung des erfindungsgemäßen Verbundwerkstoffs in einem gewissen Umfang durch die Phasenumwandlung des Zirkonoxids auftretenden lokalen Dehnungen bzw. Verzerrungen unterstützt werden, die durch einen starren Aluminiumoxid- oder Zirkonoxid-Kristall unterbunden wären. Dies wird erfindungsgemäß insbesondere dadurch erreicht, dass die erfindungsgemäß eingesetzten Dispersoide eine lokale Scherdeformation, bzw. inelastische Mikrodeformation, ermöglichen. Voraussetzung für diese lokale Mikrodeformation und damit für die erfindungsgemäß beabsichtigte Akkomodation ist die besondere Eigenschaft der erfindungsgemäß vorgesehenen Dispersoide. Die erfindungsgemäß vorgesehenen Dispersoide, die erfindungsgemäß vorgesehenen dispersoiden Kristalle, setzen aufgrund ihrer Kristallstruktur oder durch innere Grenzflächen einer Scher- bzw. Mikrodeformation deutlich geringeren Widerstand entgegen als die im Stand der Technik bisher eingesetzten starren Aluminiumoxid- oder Zirkonoxid-Kristalle.

Durch die Dehnungs-Akkomodation werden die inneren Spannungen und die lokale Verteilung der Phasenumwandlung des Zirkonoxids im erfindungsgemäßen Verbundwerkstoff bei der Spannungsumwandlung günstig beeinflusst, so dass effektiv ein höherer Widerstand gegen Rissausbreitung (sog. Risszähigkeit) erreicht wird.

Dieses im erfindungsgemäßen Verbundwerkstoff überraschend wirksame Prinzip und die erfindungsgemäßen Eigenschaften des Verbundwerkstoffs ist bzw. sind bisher im Stand der Technik nicht beschrieben worden.

Die Herstellung des erfindungsgemäßen Verbundwerkstoffs erfolgt mittels an sich bekannter, konventioneller Keramiktechnologie. Die wesentlichen Prozessschritte sind beispielsweise:
a) Pulvermischung gemäß vorgegebener Zusammensetzung in Wasser ansetzen, ggfls. Verwendung von Verflüssigern zur Vermeidung der Sedimentation.
b) Homogenisieren im Dissolver (schnelllaufender Rührer).
c) Mahlen in Rührwerkskugelmühle, dabei Erhöhung der spezifischen Oberfläche der Pulvermischung (= Zerkleinerung).
d) Evtl. Zugabe von organischen Bindern.
e) Sprühtrocknen, dabei entsteht ein rieselfähiges Granulat mit definierten Eigenschaften.
f) Befeuchten des Granulats mit Wasser.
g) Axial oder isostatisch pressen.
h) Spanabhebende Grünbearbeitung, dabei wird unter Berücksichtigung der Sinterschwindung weitgehend die Endkontur abgebildet.
i) Vorbrand, dabei Schwindung auf ca. 98% der theoretischen Dichte. Die noch verbleibenden Restporen sind nach außen geschlossen.
j) Heissisostatisches Pressen unter hoher Temperatur und hohem Gasdruck, dadurch praktisch vollständige Endverdichtung.
k) So genannter Weissbrand, dadurch wird das beim heissisostatischen Pressen erzeugte Ungleichgewicht der sauerstoffionen in der Keramik ausgeglichen.
l) Hartbearbeitung durch Schleifen und Polieren.
m) Tempern.

Verwendet werden kann der erfindungsgemäße Verbundwerkstoff beispielsweise zur Herstellung von Sinterformkörpern, zur Herstellung von Bauteilen mit der Fähigkeit zur Energieabsorption bei dynamischer Belastung in der Medizintechnik, zur Herstellung von Orthesen und Endoprothesen, beispielsweise zu Hüftgelenk- oder Kniegelenkimplantaten, Bohrern, beispielsweise für medizinische Anwendungen, Maschinenbaukomponenten, die tribologisch, chemisch und/oder thermisch beansprucht werden.

Die vorliegende Erfindung betrifft folglich einen Verbundwerkstoff aus Aluminiumoxid als keramische Matrix, darin dispergiertem Zirkonoxid, wobei
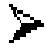 der Verbundwerkstoff als erste Phase einen Aluminiumoxidanteil von mindestens 65 Vol.-% und als zweite Phase einen Zirkonoxidanteil von 10 bis 35 Vol.% enthält und wobei das Zirkonoxid, bezogen auf den Geamtzirkonoxidgehalt, zu 80 bis 99 %, bevorzugt zu 90 bis 99 %, in der tetragonalen Phase vorliegt und wobei die Stabilisierung der tetragonalen Phase des Zirkonoxids zum überwiegenden Teil nicht chemisch sondern mechanisch erfolgt, mit einem Gesamtanteil an chemischen Stabilisatoren < 0,2 Mol%t, wobei als Nebenkomponente eine weitere Phase mit Dispersoiden (dispersoide Phase) enthalten ist, und wobei Dispersoide Platelets sind, die inelastische Mikrodeformationen auf mikroskopische Ebene ermöglichen.

Besonders bevorzugt ist ein erfindungsgemäßer Verbundwerkstoff, bei dem
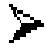 die Zirkonoxidpartikel eine Korngröße von durchschnittlich 0,1 bis 0,5 µm, bevorzugt von durchschnittlich 0,15 bis 0,25 µm aufweisen;
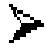 der Gehalt an chemischen Stabilisatoren relativ zum Zirkonoxid auf Werte begrenzt ist, die deutlich unterhalb der im Stand der Technik für die jeweilig verwendeten chemischen Stabilisatoren liegen;
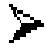 der Verbundwerkstoff keinen chemischen Stabilisator enthält;
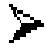 das Aluminiumoxid und/oder das Zirkonoxid lösliche Bestandteile enthält;
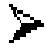 als lösliche Bestandteile im Aluminiumoxid und/oder im Zirkonoxid ein oder mehrere der folgenden Elemente vorliegen: Cr, Fe, Mg, Ti, Y, Ce, Ca, Lanthanide und/oder V;
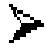 Dispersoide Platelets ermöglichen infolge ihrer Kristallstruktur Scherdeformationen auf mikroskopischer Ebene;
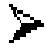 die Partikelgrößen der Dispersoide in der dispersoiden Phase deutlich größer sind als die Korngrößen des Aluminiumoxids bzw. Zirkonoxids;
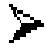 die Partikelgrößen der Dispersoide vorzugsweise 1 bis 5 µm betragen;
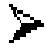 der Volumenanteil der die dispersoide Phase bildenden Dispersoide deutlich geringer ist als der Anteil des Zirkonoxids;
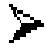 der Volumenanteil der die dispersoide Phase bildenden Dispersoide bis zu 10 Vol.-%, vorzugsweise 2 bis 8 Vol.-%, besonders bevorzugt 3 bis 6 Vol.-% beträgt;
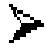 der Gehalt an die dispersoide Phase bildenden Dispersoide 2 bis 30 Millimol (mmol) pro 100 g Gesamtmasse beträgt;
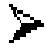 als Dispersoide Substanzen verwendet werden, die chemisch stabil sind und während der Herstellung des Verbundwerkstoffs durch Sintern bei hohen Temperaturen nicht im Aluminiumoxid oder im Zirkonoxid in Lösung gehen;
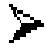 als Dispersoide Strontiumaluminat (SrAl₁₂O₁₉) oder Lanthanaluminat (LaAl₁₁O₁₈) eingesetzt werden;
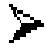 die Bruchfestigkeit >1300 MPa beträgt.

Weiterhin betrifft die vorliegende Erfindung die Verwendung des erfindungsgemäßen Verbundwerkstoffs
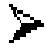 zur Herstellung von Sinterformkörpern;
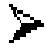 zur Herstellung von Bauteilen mit der Fähigkeit zur Energieabsorption bei dynamischer Belastung;
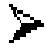 in der Medizintechnik;
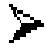 zur Herstellung von künstlichen Prothesen in der Medizintechnik, beispielsweise zur Herstellung von Orthesen und Endoprothesen;
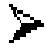 zur Herstellung von Hüftgelenk- und Kniegelenkimplantaten.

Nachfolgend wird die vorliegende Erfindung anhand von Versuchsreihen erläutert, ohne sie dadurch einzuschränken:

### Versuchsreihe 1: Risszähigkeit in Abhängigkeit des Plateletbildners

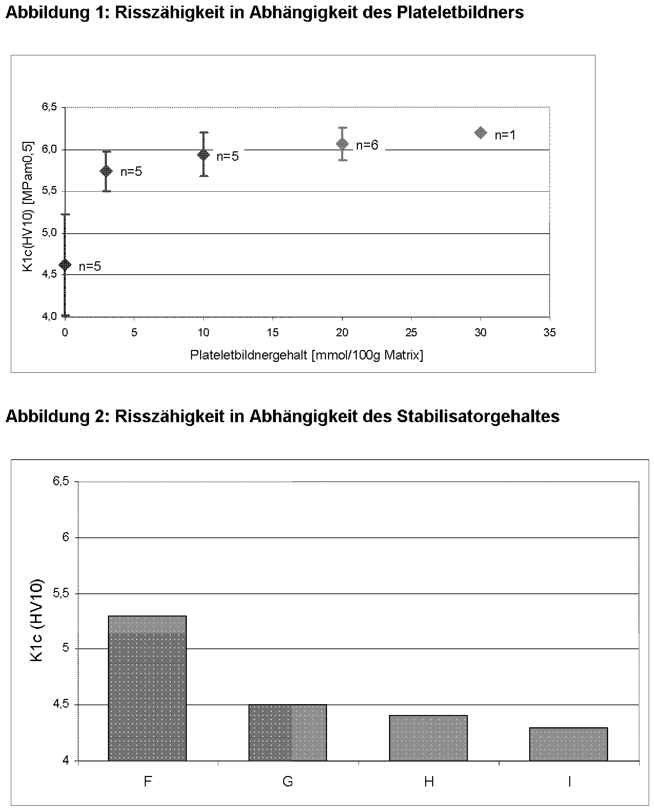

Abbildung 1 zeigt die Ergebnisse einer Versuchsreihe mit unterschiedlichen Gehalten an erfindungsgemäßen Dispersoiden. Der Dispersoidbildner ist in diesem Fall Strontium, Angabe erfolgt in Millimol (mmol) pro 100 g Gesamtmasse. Es wurden in jedem Einzelfall verschiedene Arten der Aufbereitung getestet, beispielsweise unterschiedlich lange Mahldauer oder weitere lösliche Additive. Die Anzahl der Einzelversuche je Dispersoidgehalt ist in Abbildung 1 mit der Nummer n gekennzeichnet.

Dargestellt ist die erhaltene Risszähigkeit anhand der Messung mittels Vickers-Indentereindruck (HV10). Die Darstellung zeigt deutlich, dass die Risszähigkeit ohne Dispersoide (= Platelet-Bildnergehalt Null) erheblich niedriger ist als die Risszähigkeit bei höheren Gehalten an Dispersoiden. Bei dieser Versuchsreihe wurde die höchste Risszähigkeit bei einem Gehalt von 30 mmol/100g Matrix erzielt. Eine nennenswerte Steigerung der Risszähigkeit ergab sich aber bereits bei sehr niedrigen Gehalten an Dispersoiden.

### Versuchsreihe 2: Risszähigkeit in Abhängigkeit des Stabilisatorgehaltes

Abbildung 2 zeigt die Ergebnisse einer Versuchsreihe, in der durch Reduzierung des chemischen Stabilisators eine Erhöhung der Risszähigkeit erreicht wurde. In der Abbildung sind die Risszähigkeiten unterschiedlicher Rezepturen, in der Abbildung mit F - I bezeichnet, dargestellt. Allen Rezepturen gemeinsam sind die Hauptkomponenten Al₂O₃ und ZrO₂ (21 Gew.-%). Die Rezepturen unterscheiden sich durch die Art und die Menge des chemischen Stabilisators: F → kein Stabilisator, G → 1 mol% Y₂O₃, H → 5 mol% CeO₂, I → 10 mol% CeO₂. Die Angaben für die Stabilisatoren sind relativ zum Zirkonoxidgehalt angegeben. Ce und Y wirken bekanntermaßen als chemische Stabilisatoren für die tetragonale Phase des Zirkonoxids. Es zeigt sich deutlich, dass jede Art der Stabilisatorzugabe die Risszähigkeit des Materials signifikant absenkt.

### Versuchsreihe 3: Variationen in Korngröße und Stabilisierung

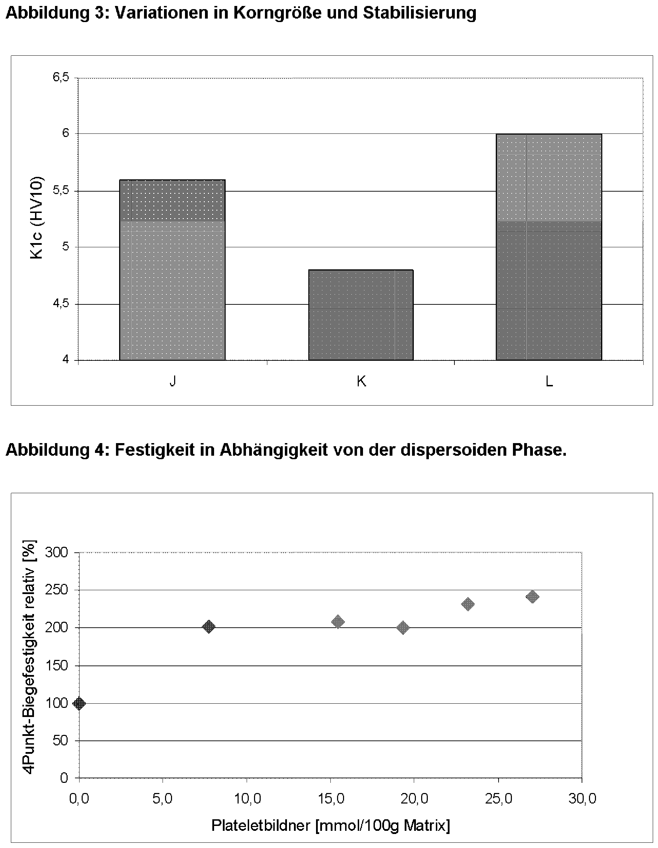

Abbildung 3 zeigt die Ergebnisse einer Versuchsreihe, in der die Wechselwirkung aus chemischer Stabilisierung und Gefüge untersucht wurde. Die in Abbildung 3 mit J, K und L bezeichneten Werkstoffe sind wie folgt gekennzeichnet:
J = ZTA mit 24 Gew.-% ZrO₂, 3 Gew.-% SrAl₁₂O₁₉ und 1,3 mol% Y₂O₃; die Korngröße des Zirkonoxids beträgt 0,3 µm.

K = J, die Korngröße des Zirkonoxids beträgt jedoch 0,2 µm. Offensichtlich wird die Risszähigkeit durch Verringerung der Zirkonoxid-Korngröße erheblich reduziert, gleichbedeutend mit mechanischer Überstabilisierung.

L = J, jedoch mit halbiertem Y₂O₃-Gehalt. Die mechanische Überstabilisierung wurde durch die reduzierte chemische Stabilisierung wieder ausgeglichen, dadurch wurde die Risszähigkeit wieder deutlich erhöht.

### Versuchsreihe 4: Festigkeit in Abhängigkeit von der dispersoiden Phase.

Abbildung 4 zeigt die Ergebnisse einer Versuchsreihe, in der die Festigkeit der erfindungsgemäßen Verbundwerkstoffe in Abhängigkeit vom Gehalt an dispersoider Phase in der Matrix untersucht wurde. Die Zugabe von Plateletbildenden Oxiden, in dieser Versuchsreihe SrAl₁₂O₁₉, führt zu einer signifikanten Steigerung der 4-Punkt-Biegefestigkeit. Der größte Sprung in der Festigkeit zeigt sich analog zur Risszähigkeit zwischen 0 und 10 mmol/100g Matrix. Die weitere Erhöhung auf bis zu 27 mmol/100g Matrix ergibt einen weiteren leichten Anstieg der Festigkeit. Durch Zugabe der erfindungsgemäßen Dispersoide werden dadurch Festigkeiten bis über 1.300 MPa erreicht, Festigkeiten die ohne Dispersoide nicht zu erreichen sind.

### Versuchsreihe 5: Auswirkungen der chemischen Stabilisierung auf hydrothermale Alterung

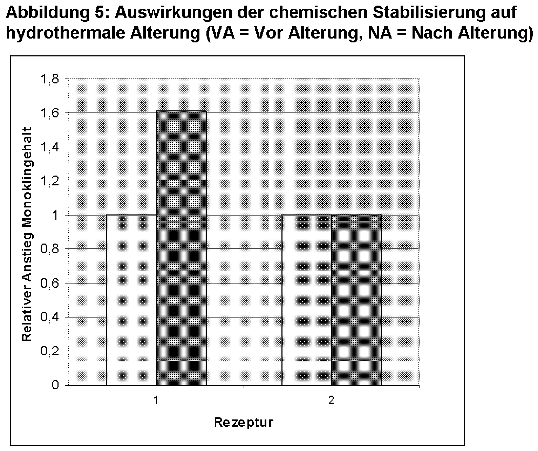

Abbildung 5 zeigt die Ergebnisse einer Versuchsreihe, in der die Auswirkungen der chemischen Stabilisierung auf die hydrothermale Alterung untersucht wurden.

Die Abbildung zeigt die Ergebnisse eines Versuchs zur hydrothermalen Alterung (VA = Vor Alterung, NA = Nach Alterung), also die Zunahme der monoklinen Phase bei Auslagerung in Wasserdampf. Gemäß Normentwurf ISO/DIS 6474-2 wurden folgende Bedingungen gewählt. Wasserdampf, 0,2 MPa Druck, 134°C, 10h. Die Rezepturen 1 und 2 unterscheiden sich nur im Yttriumoxid-Gehalt.
Rezeptur 1 : 1,3 mol% Yttriumoxid
Rezeptur 2 : 0,0 mol% Yttriumoxid

Im Ausgangszustand weisen beide Sorten einen Monoklingehalt von < 10% auf. Zur besseren Unterscheidbarkeit wurde der Monoklingehalt in Abbildung 5 normiert.

Rezeptur 1 zeigt einen relativen Anstieg des Monoklingehaltes von 60%, wohingegen Rezeptur 2 keinerlei Veränderung des Monoklingehaltes zeigt. Damit ist nachgewiesen, dass die erfindungsgemäße Lehre, Verzicht auf chemische Stabilisierung, zu einer wesentlichen Verbesserung im Hinblick auf die Beständigkeit des erfindungsgemäßen Verbundwerkstoffs gegen hydrothermale Alterung führt.

## Patentansprüche

1. Verbundwerkstoff aus Aluminiumoxid als keramische Matrix mit darin dispergiertem Zirkonoxid, **dadurch gekennzeichnet, dass** der Verbundwerkstoff als erste Phase einen Aluminiumoxidanteil von mindestens 65 Vol.-% und als zweite Phase einen Zirkonoxidanteil von 10 bis 35 Vol.-% enthält, wobei das Zirkonoxid, bezogen auf den Geamtzirkonoxidgehalt zu 80 bis 99 %in der tetragonalen Phase vorliegt, wobei der Gesamtgehalt an chemischen Stabilisatoren < 0,2 Mol% beträgt, so dass die Stabilisierung der tetragonalen Phase des Zirkonoxids zum überwiegenden Teil nicht chemisch sondern mechanisch erfolgt, und wobei zusätzlich als Nebenkomponente eine weitere Phase mit Dispersoiden (dispersoide Phase) enthalten ist, wobei Dispersoide Platelets sind, die inelastische Mikrodeformationen auf mikroskopischer ebene ermöglichten.

2. Verbundwerkstoff gemäß Anspruch 1, **dadurch gekennzeichnet, dass** das Zirkonoxid bezogen auf den Gesamtzirkonoxidgehalt zu 90 bis 99 % in der tetragonalen Phase vorliegt.

3. Verbundwerkstoff gemäß Anspruch 1 oder 2, **dadurch gekennzeichnet, dass** die Zirkonoxidpartikel eine Korngröße von durchschnittlich 0,1 bis 0,5 µm, bevorzugt von durchschnittlich 0,15 bis 0,25 µm aufweisen.

4. Verbundwerkstoff gemäß einem oder mehreren der vorhergehenden Ansprüche, **dadurch gekennzeichnet, dass** der Verbundwerkstoff keinen chemischen Stabilisator enthält.

5. Verbundwerkstoff gemäß Anspruch 1, **dadurch gekennzeichnet, dass** die Platelets infolge ihrer Kristallstruktur Scherdeformationen auf mikroskopischer Ebene ermöglichen.

6. Verbundwerkstoff gemäß einem der Ansprüche 1 oder 5, **dadurch gekennzeichnet, dass** die Partikelgrößen der Platelets in der dispersoiden Phase deutlich größer sind als die Korngrößen des Aluminiumoxids bzw. Zirkonoxids und vorzugsweise eine Partikelgröße von 1 bis 5 µm aufweisen.

7. Verbundwerkstoff gemäß einem der Ansprüche 1 oder 5und 6, **dadurch gekennzeichnet, dass** der Volumenanteil der die dispersoide Phase bildenden Dispersoide deutlich geringer ist als der Anteil des Zirkonoxids und bevorzugt bis zu 10 Vol.-%, besonders bevorzugt 2 bis 8 Vol.-% und insbesondere bevorzugt 3 bis 6 Vol.-% beträgt.

8. Verbundwerkstoff gemäß einem der Ansprüche 1 und 5 bis 7, **dadurch gekennzeichnet, dass** der Gehalt an die dispersoide Phase bildenden Dispersoide 2 bis 30 Millimol (mmol) pro 100 g Gesamtmasse beträgt.

9. Verbundwerkstoff gemäß einem der Ansprüche 1 und 8 bis 11, **dadurch gekennzeichnet, dass** als Dispersoide Strontiumaluminat SrAl₁₂O₁₉ oder Lanthanaluminat LaAl₁₁O₁₈ eingesetzt werden.

10. Verbundwerkstoff gemäß einem oder mehreren der vorhergehenden Ansprüche, **dadurch gekennzeichnet, dass** die Bruchfestigkeit >1300 MPa beträgt.

11. Verwendung des Verbundwerkstoffs gemäß einem oder mehreren der Ansprüche 1 bis 10 zur Herstellung von Sinterformkörpern.

12. Verwendung des Verbundwerkstoffs gemäß einem oder mehreren der Ansprüche 1 bis 10 zur Herstellung von Bauteilen mit der Fähigkeit zur Energieabsorption bei dynamischer Belastung oder in der Medizintechnik, insbesondere zur Herstellung von künstlichen Prothesen, beispielsweise Orthesen und Endoprothesen und besonders bevorzugt zur Herstellung von Hüftgelenk- und Kniegelenkimplantaten.

## Claims

1. A composite material of aluminium oxide as a ceramic matrix with zirconium oxide dispersed therein, **characterised in that** the composite material contains as a first phase a proportion of aluminium oxide of at least 65 % by volume and as a second phase a proportion of zirconium oxide of 10 to 35 % by volume, wherein 80 to 99 % of the zirconium oxide, relative to the total zirconium oxide content, is present in the tetragonal phase, wherein the total content of chemical stabilizers amounts to < 0.2 mol % so that the stabilization of the tetragonal phase of the zirconium oxide for the most part is not effected chemically, but mechanically, and wherein there is additionally contained as a subsidiary component a further phase with dispersoids (dispersoid phase), wherein dispersoids are platelets that render possible inelastic micro-deformations on a microscopic level.

2. A composite material according to claim 1, **characterised in that** 90 to 99 % of the zirconium oxide, relative to the total zirconium oxide content, is present in the tetragonal phase.

3. A composite material according to claim 1 or 2, **characterised in that** the zirconium oxide particles have a grain size of on average 0.1 to 0.5 µm, preferably of on average 0.15 to 0.25 µm.

4. A composite material according to one or more of the preceding claims, **characterised in that** the composite material does not contain a chemical stabilizer.

5. A composite material according to claim 1, **characterised in that** the platelets, as a result of their crystal structure, render possible shear deformations on a microscopic level.

6. A composite material according to one of claims 1 or 5, **characterised in that** the particle sizes of the platelets in the dispersoid phase are significantly larger than the grain sizes of the aluminium oxide or zirconium oxide and preferably have a particle size of 1 to 5 µm.

7. A composite material according to one of claims 1 or 5 and 6, **characterised in that** the volume proportion of the dispersoids forming the dispersoid phase is significantly lower than the proportion of the zirconium oxide and preferably amounts to up to 10 % by volume, particularly preferably 2 to 8 % by volume, and in particular preferably 3 to 6 % by volume.

8. A composite material according to one of claims 1 and 5 to 7, **characterised in that** the content of dispersoids forming the dispersoid phase amounts to 2 to 30 millimoles (mmol) per 100 g total mass.

9. A composite material according to one of claims 1 and 8 to 11, **characterised in that** strontium aluminate SrAl₁₂O₁₉ or lanthanum aluminate LaAl₁₁O₁₈ are used as dispersoids.

10. A composite material according to one or more of the preceding claims, **characterised in that** the breaking strength amounts to > 1300 MPa.

11. Use of the composite material according to one or more of claims 1 to 10 for the production of sintered moulded bodies.

12. Use of the composite material according to one or more of claims 1 to 10 for the production of components having the ability to absorb energy under dynamic load or in medical technology, in particular for the production of artificial prostheses, for example or theses and endoprostheses, and particularly preferably for the production of hip-joint and knee-joint implants.

## Revendications

1. Matériau composite à base d'oxyde d'aluminium en tant que matrice céramique avec de l'oxyde de zirconium dispersé dans celle-ci, **caractérisé en ce que** le matériau composite contient en tant que première phase une proportion d'oxyde d'aluminium d'au moins 65 % en volume et en tant que deuxième phase une proportion d'oxyde de zirconium de 10 à 35 % en volume, l'oxyde de zirconium se trouvant dans la phase tétragonale à raison de 80 à 99 %, par rapport à la teneur totale en oxyde de zirconium, la teneur totale en stabilisants chimiques étant < 0,2 % en moles, de sorte que la stabilisation de la phase tétragonale de l'oxyde de zirconium en la fraction prépondérante s'effectue non pas chimiquement mais mécaniquement, et en outre une autre phase comportant des dispersoïdes (phase dispersoïde) étant contenue en tant que composante secondaire, les dispersoïdes étant des plaquettes qui permettent des microdéformations inélastiques sur le plan microscopique.

2. Matériau composite selon la revendication 1, **caractérisé en ce que** l'oxyde de zirconium est présent dans la phase tétragonale à raison de 90 à 99 %, par rapport à la teneur totale en oxyde de zirconium.

3. Matériau composite selon la revendication 1 ou 2, **caractérisé en ce que** les particules d'oxyde de zirconium présentent une taille de grain d'en moyenne 0,1 à 0,5 µm, de préférence d'en moyenne 0,15 à 0,25 µm.

4. Matériau composite selon une ou plusieurs des revendications précédentes, **caractérisé en ce que** le matériau composite ne contient pas de stabilisant chimique.

5. Matériau composite selon la revendication 1, **caractérisé en ce qu'**en raison de leur structure cristalline les plaquettes permettent des déformations de cisaillement sur le plan microscopique.

6. Matériau composite selon l'une quelconque des revendications 1 et 5, **caractérisé en ce que** les tailles de particules des plaquettes dans la phase dispersoïde sont nettement plus grandes que les tailles de particules de l'oxyde d'aluminium ou de l'oxyde de zirconium et de préférence les particules présentent une taille de particule de 1 à 5 µm.

7. Matériau composite selon l'une quelconque des revendications 1 et 5 et 6, **caractérisé en ce que** la proportion en volume des dispersoïdes formant la phase dispersoïde est nettement plus faible que la proportion de l'oxyde de zirconium et de préférence vaut jusqu'à 10 % en volume, de façon particulièrement préférée de 2 à 8 % en volume et de façon particulièrement préférée de 3 à 6 % en volume.

8. Matériau composite selon l'une quelconque des revendications 1 et 5 à 7, **caractérisé en ce que** la teneur en dispersoïdes formant la phase dispersoïde vaut de 2 à 30 millimoles (mmoles) pour 100 g de masse totale.

9. Matériau composite selon l'une quelconque des revendications 1 et 8 à 11, **caractérisé en ce qu'**on utilise comme dispersoïdes de l'aluminate de strontium SrAl₁₂O₁₉ ou de l'aluminate de lanthane LaAl₁₁O₁₈.

10. Matériau composite selon une ou plusieurs des revendications précédentes, **caractérisé en ce que** la résistance à la rupture vaut > 1 300 MPa.

11. Utilisation du matériau composite selon une ou plusieurs des revendications 1 à 10, pour la production de corps moulés frittés.

12. Utilisation du matériau composite selon une ou plusieurs des revendications 1 à 10, pour la production de pièces de construction ayant la capacité de l'absorption d'énergie sous contrainte dynamique ou dans la technique médicale, en particulier pour la fabrication de prothèses artificielles, par exemple d'orthèses et d'endoprothèses et de façon particulièrement préférée pour la fabrication d'implants d'articulations du genou et de la hanche.
